# EUROPÄISCHE PATENTSCHRIFT

(11) **EP 2 779 931 B1**
(45) Veröffentlichungstag und Bekanntmachung des Hinweises auf die Patenterteilung: **29.03.2017**
(21) Anmeldenummer: 12797700.7
(22) Anmeldetag: 15.11.2012
(51) Int. Cl.: A61B 18/22, G02B 6/02

(54) **VORRICHTUNG ZUR MEDIZINISCHEN BEHANDLUNG, INSBESONDERE EINES GEWEBES, MITTELS LASERLICHT**
DEVICE FOR MEDICAL TREATMENT, MORE PARTICULARLY OF A TISSUE, BY MEANS OF LASER LIGHT
DISPOSITIF DE TRAITEMENT MÉDICAL, EN PARTICULIER D'UN TISSU, AU MOYEN D'UNE LUMIÈRE LASER

(30) Priorität: 18.11.2011 US 201161561584 P; 19.11.2011 DE 102011118875
(43) Veröffentlichungstag der Anmeldung: 24.09.2014
(73) Patentinhaber: Advanced Fiber Tools GmbH, 09648 Mittweida (DE)
(72) Erfinder: KUKA, Georg, 12555 Berlin (DE); ASHRAF, Naim, 53123 Bonn (DE); HÄHNEL, Torsten, 09117 Chemnitz (DE)
(86) Internationale Anmeldenummer: PCT/EP2012/004746
(87) Internationale Veröffentlichungsnummer: WO 2013/072057

(56) Entgegenhaltungen:
- US-A- 5 320 620
- US-A- 5 495 541
- US-A1- 2010 262 131
- US-B2- 7 916 991

## Beschreibung

Die Erfindung betrifft eine Vorrichtung zur medizinischen Behandlung, insbesondere eines Gewebes, mittels Laserlichts gemäß dem Oberbegriff des Anspruchs 1 und ein Verfahren zur Herstellung der Vorrichtung gemäß dem Oberbegriff des Anspruchs 6.

Applikatoren zur medizinischen Behandlung von lebendem Gewebe mit Hilfe von Lasern sind bekannt. Insbesondere ist es bekannt, Prostatagewebe mittels Laserlichts operativ zu entfernen oder volumenmäßig zu reduzieren. Als Lichtquelle kommen dabei heute vorzugsweise Holmiumlaser zum Einsatz, die bei einer Emissionswellenlänge (Lichtwellenlänge) im Mikrometerbereich (ca. 2123 nm) hohe Lichtleistungen erzeugen, was eine effektive Gewebeabtragung ermöglicht. Aufgrund seiner Lichtwellenlänge wird das Licht des Holmiumlasers stark vom Wasser absorbiert, wobei das wasserhaltige Prostatagewebe bei Bestrahlung verdampft. Die geringe Eindringtiefe ins Gewebe von unter einem Millimeter führt zu sehr dünnen Schnitten.

Das Laserlicht wird an einem Ende in eine Multimode-Lichtleit-Faser eingekoppelt und am anderen Ende stirnseitig wieder ausgekoppelt. Zur Lichtleitung weist die Lichtleit-Faser einen zentralen Kern auf, der meist aus Quarzglas besteht. Über dem Kern ist mindestens ein Cladding (Glasmantel) und über diesem zum Schutz ein Kunststoffmantel angeordnet. Die Lichtleit-Faser endet meist in einem distalen Endstück.

Aus der US2010262131A1 und der US5495541 ist bereits ein Applikator zur medizinischen Behandlung eines Gewebes mittels Laserlichts bekannt, bei dem das Laserlicht in eine optische Lichtleit-Faser eingekoppelt und zum Lichtaustrittsende geleitet wird. Die Lichtleit-Faser weist einen Kern und ein darüber angeordnetes Cladding auf. Das Lichtaustrittsende ist jeweils hakenförmig gekrümmt, so dass das Laserlicht im wesentlichen quer zu dem vor der Krümmung (Biegung) liegenden Längenabschnitt stirnseitig aus dem Lichtaustrittsende austritt und auf das Gewebe trifft.

In der US5495541 ist die numerische Apertur des gekrümmten Lichtaustrittsendes jeweils größer als die numerische Apertur des Laserlichts in der geraden Lichtleit-Faser, um Verluste durch seitlich austretendes Licht im Krümmungsbereich (Biegebereich) zu verhindern. Zur Vergrößerung der numerischen Apertur des Lichtaustrittsendes wird das Cladding im Krümmungsbereich entfernt und durch ein Material mit kleinerer Brechzahl ersetzt. Speziell ist das gekrümmten Lichtaustrittsende ohne Cladding in einem verschlossenen Röhrchen angeordnet und dort in ein Material mit einer Brechzahl von ungefähr 1 eingebettet, was eine relativ große numerische Apertur des gekrümmten Lichtaustrittsendes bewirkt und dadurch einen relativ großen Krümmungswinkel ermöglicht. Das Medium übernimmt dabei die Funktion des Claddings. Durch die Einbettung des Krümmungsbereichs besteht die Lichtleit-Faser physikalisch gesehen aus zwei Lichtleit-Fasern mit unterschiedlicher Apertur, die sich unmittelbar aneinander anschließen.

Nachteilig ist dabei, dass das Entfernen des Claddings sehr aufwendig ist, insbesondere bei Lichtleit-Fasern mit einem Cladding aus Glas.

Die US7916991B2 zeigt weiter eine Lichtleit-Faser, die einen Kern mit einem inneren Cladding aufweist, über dem ein erstes äußeres Cladding angeordnet in, in dem radial beabstandete Kapillaren in Faserlängsrichtung verlaufen, die im Querschnitt gesehen einen Kapillarring bilden. In einem geraden Längenabschnitt (longitudinalen Bereich) ist die Lichtleit-Faser soweit radial verjüngt, dass der verjüngte Bereich frei von Kapillaren ist. Der verjüngte Bereich ist in einen Absorber eingebettet.

Ferner ist aus der US5320620 eine medizinischen Behandlungsvorrichtung mit einer Lichtleit-Faser bekannt, die in einem Endstück mündet, das eine gekrümmte Austrittsfläche aufweist, damit das Laserlicht auf einer Seite aus dem Endstück austritt. Diese Lösung hat allerdings den Nachteil, dass immer noch ein größerer Teil des Laserlichts durch die gekrümmte Außenfläche hindurch austritt, was bei einer Wellenlänge des Laserlichts im Mikrometerbereich wie beim Holmiumlaser zu unerwünschten Schäden am umliegenden Gewebe führen würde.

Die Aufgabe der Erfindung ist es, eine technisch einfach herzustellende Vorrichtung (Applikator) zur medizinischen Behandlung, insbesondere zur Abtragung und Reduzierung eines lebenden Gewebes, mittels einer Lichtleit-Faser vorzuschlagen, deren Cladding insbesondere aus Glas besteht und bei der das Laserlicht nahezu verlustfrei in einer Richtung quer zu der im Wesentlichen geraden Lichtleit-Faser austritt.

Die Aufgabe wird bezogen auf die Vorrichtung durch die Merkmale des Anspruchs 1 und bezogen auf das Verfahren durch die Merkmale des Anspruchs 6 gelöst; die Unteransprüche stellen vorteilhafte Ausgestaltungen dar.

Die Lösung sieht bezogen auf die Vorrichtung vor, dass im Mantel in Faserlängsrichtung verlaufende Kapillaren in einem radialen Abstand von der Längsachse der Lichtleit-Faser angeordnet sind, die im Querschnitt gesehen einen Kapillarring bilden, dessen Kapillarhohlräume durch Stege voneinander getrennt sind, deren Breite kleiner als die Lichtwellenlänge des Laserlichts ist, und dass die Kapillaren am Lichtaustrittsende der Lichtleit-Faser durch Schmelzen der Lichtleit-Faser verschlossen sind und das Lichtaustrittsende hakenförmig gebogen ist. Die Kapillaren (der Kapillarring) sorgen dabei für eine starke Lichtführung in der Faser. Die parallel zum Kern verlaufenden Kapillaren führen bei Krümmung des Lichtaustrittsendes bis zu einem relativ großen Krümmungswinkel von ca. 60 Grad dazu, dass das Laserlicht nur an der Stirnseite austritt, wenn deren Abstand (die Stegbreite) hinreichend klein ist.

Technisch einfach ist es, wenn das Lichtaustrittsende der Lichtleit-Faser mit der Krümmung endet, d.h. mit der Krümmung ausläuft.

Der Lichtaustritt lässt sich verbessern, wenn an der Krümmung lichtaustrittsseitig ein distales Endstück angeordnet ist, so dass das Laserlicht aus dem Endstück austritt. Das Endstück ist der Stirnseite der entsprechenden Lichtleit-Faser unmittelbar nachgeschaltet.

Zweckmäßigerweise sind die Kapillaren verschlossen, insbesondere durch das distale Endstück.

Eine einfache und technisch stabile Ausbildung sieht vor, dass die Austrittsfläche des distalen Endstücks durch Schleifen und Polieren gebildet ist.

Die Lichtführung im Bereich der Krümmung lässt sich verbessern, wenn die Lichtleit-Faser mehrere Kapillarringe aufweist.

Die Lösung sieht bezogen auf Verfahren zur Herstellung einer gekrümmten optischen Lichtleit-Faser vor, dass eine Lichtleit-Faser verwendet wird, die im Cladding in Faserlängsrichtung verlaufende Kapillaren in einem radialen Abstand von der Längsachse der Lichtleit-Faser aufweist, die im Querschnitt gesehen einen Kapillarring bilden, dessen Kapillarhohlräume durch Stege voneinander getrennt sind, deren Breite kleiner als die Lichtwellenlänge des Laserlichts ist, dass die Kapillaren durch Schmelzen des Lichtaustrittsendes verschlossen werden und dass danach das Lichtaustrittsende mit den verschlossenen Kapillaren hakenförmig gebogen wird.

Die Erfindung wird nachfolgend anhand eines Ausführungsbeispiels näher beschrieben. Es zeigen:
- Fig. 1: ein Applikator mit einem Holmiumlaser in einer schematischen Darstellung,
- Fig. 2: das Lichtaustrittsende zusammen mit dem davor liegenden geraden Längenabschnitt,
- Fig. 3: einen Querschnitt durch die Lichtleit-Faser,
- Fig. 4: einen Ausschnitt des Querschnitts gemäß Fig. 3 und
- Fig. 5: einen Ausschnitt des Ausschnitts gemäß Fig. 4.

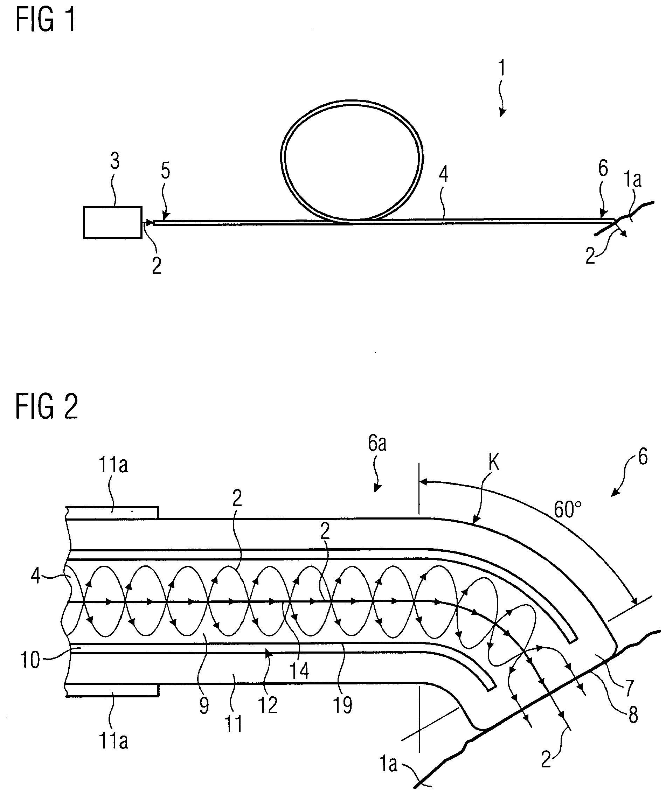

Fig. 1 zeigt in einer schematischen Darstellung einen Applikator 1 (Behandlungsvorrichtung) zur medizinischen Behandlung, insbesondere von lebendem Prostatagewebe 1a. Die Behandlung erfolgt mit Hilfe von Laserlicht 2, das von einem Holmiumlaser 3 erzeugt wird. Das Laserlicht 2 wird zur Behandlungsstelle mittels einer mikrostrukturierten Multimode-Lichtleit-Faser 4 mit einer numerischen Apertur (NA) von 0,6 geleitet, wobei es in bekannter Weise (hier nur schematisch angedeutet) an einem Ende 5 der Lichtleit-Faser 4 in diese eingekoppelt wird und am anderen Ende, dem Lichtaustrittsende 6, stirnseitig aus dieser wieder austritt. Die Austrittsrichtung ist dabei die Richtung, der die zugehörige Stirnseite des Lichtaustrittsendes 6 zugewandt ist.

In Fig. 2 ist das Lichtaustrittsende 6 zusammen mit dem unmittelbar davor liegenden (im Wesentlichen) geraden Längenabschnitt 6a der mikrostrukturierten Lichtleit-Faser 4 schematisch dargestellt. Die Lichtleit-Faser 4 weist am Ende eine Hakenform auf, wobei der Längenabschnitt 6a den Hakenschaft und das Lichtaustrittsende 6 das gebogene und dabei quer abragende Hakenteil bildet. Der Hakenschaft ist gerade bezogen auf die gebogene Form (die Biegung) des Hakenteils.

Die Lichtleit-Faser 4 weist einen Kern 9 aus Quarzglas auf, über dem sich ein Cladding 10 (Mantel) befindet. Das Cladding 10 ist hier nochmals mit Quarzglas 11 überfangen, um die Stabilität des Lichtaustrittsendes 6 einschließlich des unmittelbar davor liegenden Längenabschnitts 6a (des distalen Endes) zu erhöhen. Das Quarzglas 11 ist mit einem Kunststoffmantel 11a überzogen, der sich allerdings nicht bis zum Lichtaustrittsende 6 erstreckt.

Das eingekoppelte Laserlicht 2 bewegt sich im Wesentlichen durch den Kern 9 der Lichtleit-Faser 4 zu deren Lichtaustrittsende 6. Das Cladding 10 weist in Faserlängsrichtung verlaufende nebeneinander liegende Kapillaren 12 auf, die jeweils in einem radialen Abstand von der (Mantel-)Außenfläche 19 des Kerns 9 bzw. von der Längsachse 14 der Lichtleit-Faser 4 angeordnet sind.

Das Lichtaustrittsende 6 ist gegenüber dem Längenabschnitt 6a um einen Winkel von hier 60 Grad mit einem relativ kleinen Biegeradius gebogen, also mit einer Krümmung K versehen, so dass die Richtung des austretenden Laserlichts 2 quer zur Längsrichtung des geraden Längenabschnitts 6a verläuft, also quer zu dem unmittelbar vor der Krümmung K liegenden (ungekrümmten) Bereich der Lichtleit-Faser 4. Die Krümmung K lässt sich durch Biegen des Endes 6 nach Erwärmen z.B. in einem Lichtbogen herstellen, wobei dafür zu sorgen ist, dass die Kapillaren 12 nicht kollabieren.

An die Lichtleit-Faser 4 schließt sich unmittelbar ein distales Endstück 7 an und bildet mit dieser zusammen das Lichtaustrittsende 6, das durch Schmelzen der Lichtleit-Faser 4 am Faserende und anschließendes Schleifen und Polieren hergestellt wird. Vor dem Schmelzen der Lichtleit-Faser 4 muss der Kunststoffmantel 11a am Lichtaustrittsende 6 entfernt werden. Das Laserlicht 2 tritt stirnseitig aus dem Endstück 7 aus, durch welches das Lichtaustrittsende 6 quasi etwas verlängert ist. Das Endstück 7 kann selbstverständlich auch andere Endstück-Konfigurationen als in Fig. 2 dargestellt aufweisen. Die Richtung des aus der Stirnseite des Endstücks 7 austretenden Laserlichts 2 ist im Wesentlichen senkrecht zur Stirnseiten-Austrittsfläche 8. Ohne Endstück 7 würde das Laserlicht 2 direkt aus der Stirnseite der Lichtleit-Faser 4 austreten, was grundsätzlich auch möglich ist.

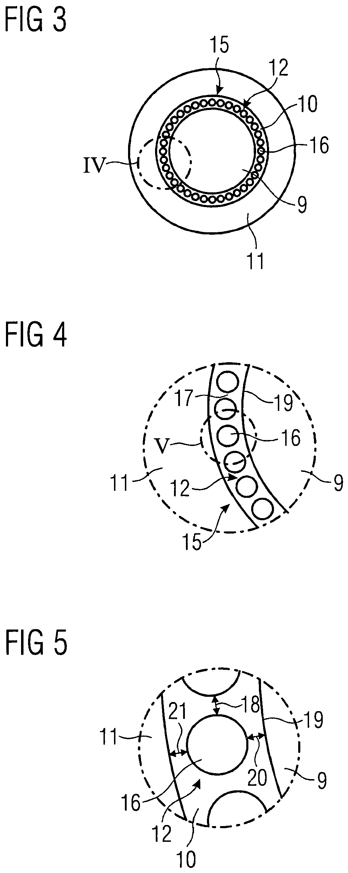

Fig. 3 zeigt einen Querschnitt durch die Lichtleit-Faser 4. Die Kapillaren 12 bilden im Querschnitt gesehen um die Längsachse 14 der Lichtleit-Faser 4 einen Ring (Kapillarring 15) von nebeneinander liegenden Hohlräumen (Kapillarhohlräume 16). Grundsätzlich können auch mehrere Kapillarringe 15 vorhanden sein.

Der Ausschnitt IV des Kapillarrings 15 von Fig. 3 ist in Fig. 4 vergrößert dargestellt. Fig. 4 zeigt, dass die Kapillarhohlräume 16 des Kapillarrings 15 durch Stege 17 voneinander getrennt sind.

In Fig. 5 ist der Ausschnitt V von Fig. 4 vergrößert dargestellt. Die Breite 18 der Stege 17 beträgt ca. 1 Mikrometer und ist damit kleiner als die Lichtwellenlänge von ca. 2 Mikrometer des Laserlichts 2. Der radiale Abstand 20 der Hohlräume 16 von der Außenfläche 19 des Kerns 9 als auch der radiale Abstand 21 von dem Quarzglas 11 ist hier etwa gleich der Stegbreite 18; sie 20, 21 können aber auch jeweils ein Vielfaches der Stegbreite 18 betragen.

Aufgrund des Kapillarrings 15 kommt es trotz der starken Krümmung K zu keinem seitlichen Lichtaustritt aus der Lichtleit-Faser 4. Die parallel zum Kern verlaufenden Kapillaren führen auch bei starker Krümmung des Lichtaustrittsendes 6 dazu, dass das Laserlicht 2 nur an der Stirnseite austritt, allerdings unter der hier erfüllten Voraussetzung, dass die Stegbreite 18 kleiner als die Lichtwellenlänge des Laserlichts 2 ist. Die Austrittsrichtung des Laserlichts 2 verläuft dadurch mit einem Winkel von 60 Grad quer zu der zumindest im Längenabschnitt 6a im Wesentlichen geraden Lichtleit-Faser 4. Üblicherweise wird die Lichtleit-Faser 4 und damit der Längenabschnitt 6a während der Behandlung in Längsrichtung des Längenabschnitts 6a verschoben als auch um seine Längsachse gedreht, wobei sich die Stirnseiten-Austrittsfläche 8 im Kontakt (Kontakt-Modus) mit dem Prostatagewebe 1a befindet.

## Patentansprüche

1. Vorrichtung zur medizinischen Behandlung mittels Laserlichts (2),
mit einer optischen Lichtleit-Faser (4), die zum Weiterleiten des eingekoppelten Laserlichts (2) einen Kern (9) und ein darüber angeordnetes Cladding (10) aufweist,
wobei das Lichtaustrittsende (6) der Lichtleit-Faser (4) gekrümmt ist und zum stirnseitigen Austritt des Laserlichts (2) aus dem Lichtaustrittsende (6) eingerichtet ist , wobei die Austrittsrichtung quer zu einem unmittelbar vor der Krümmung (K) liegenden im Wesentlichen geraden Längenabschnitt (6a) verläuft,
**dadurch gekennzeichnet,**
**dass** im Cladding (10) in Faserlängsrichtung verlaufende Kapillaren (12) in einem radialen Abstand von der Längsachse (14) der Lichtleit-Faser (4) angeordnet sind, die im Querschnitt gesehen einen Kapillarring (15) bilden, dessen Kapillarhohlräume (15) durch Stege (17) voneinander getrennt sind, deren Breite (18) kleiner als die Lichtwellenlänge des Laserlichts (2) ist, und dass die Kapillaren (12) am Lichtaustrittsende (6) der Lichtleit-Faser (4) durch Schmelzen der Lichtleit-Faser (4) verschlossen sind und das Lichtaustrittsende (6) hakenförmig gebogen ist.

2. Vorrichtung nach Anspruch 1,
**dadurch gekennzeichnet,**
**dass** an der Krümmung (K) lichtaustrittsseitig ein distales Endstück (7) angeordnet ist, aus dem das Laserlicht (2) austritt.

3. Vorrichtung nach Anspruch 2,
**dadurch gekennzeichnet,**
**dass** die Kapillaren (12) durch das distale Endstück (7) verschlossen sind.

4. Vorrichtung nach einem der Ansprüche 2 oder 3,
**dadurch gekennzeichnet,**
**dass** die Austrittsfläche des distalen Endstücks (7) durch Schleifen und Polieren gebildet ist.

5. Vorrichtung nach einem der Ansprüche 1 - 4,
**dadurch gekennzeichnet,**
**dass** die Lichtleit-Faser (4) mehrere Kapillarringe (15) aufweist.

6. Verfahren zur Herstellung einer gekrümmten optischen Lichtleit-Faser (4) für eine Vorrichtung zur medizinischen Behandlung mittels Laserlichts (2),
wobei die Lichtleit-Faser (4) zum Weiterleiten des eingekoppelten Laserlichts (2) einen Kern (9) mit einem darüber angeordneten Cladding (10) und ein Lichtaustrittsende (6) aufweist,
wobei das Laserlicht (2) stirnseitig aus dem Lichtaustrittsende (6) austritt,
bei dem das Lichtaustrittsende (6) der Lichtleit-Faser (4) derart gekrümmt wird, dass die Austrittsrichtung quer zu einem unmittelbar vor der Krümmung (K) liegenden im Wesentlichen geraden Längenabschnitt (6a) verläuft,
**dadurch gekennzeichnet,**
**dass** eine Lichtleit-Faser (4) verwendet wird, die im Cladding (10) in Faserlängsrichtung verlaufende Kapillaren (12) in einem radialen Abstand von der Längsachse (14) [der Lichtleit-Faser (4)] aufweist, die im Querschnitt gesehen einen Kapillarring (15) bilden, dessen Kapillarhohlräume (15) durch Stege (17) voneinander getrennt sind, deren Breite (18) kleiner als die Lichtwellenlänge des Laserlichts (2) ist, dass die Kapillaren (12) durch Schmelzen des Lichtaustrittsendes (6) verschlossen werden und dass danach das Lichtaustrittsende (6) mit den verschlossenen Kapillaren (12) hakenförmig gebogen wird.

## Claims

1. Device for medical treatment by means of laser light (2), using an optical light-conducting fibre (4) that has a core (9) for conducting the coupled-in laser light (2), the said core being surrounded by a cladding (10),
wherein the light-exit end (6) of the optical fibre (4) is curved and where the exit end face (6) of the laser light (2) is so aligned, wherein the exit direction is transverse to a mainly straight length (6a) that immediately precedes the bend (K), **characterised in**
**that** capillaries (12) are arranged within the cladding (10) and run in the longitudinal direction at a radial distance from the longitudinal axis (14) of the optical fibre (4), that form, seen in cross-section, a capillary ring (15) whose capillary cavities (15) are separated from each other by webs (17) whose width (18) is less than the wavelength of the laser light (2) and that the capillaries (12) at the light-exit end (6) of the optical fibre (4) are closed by melting the optical fibre (4) and that the light exit end (6) is bent into a hook-shape.

2. Device according to claim 1,
**characterised in**
**that** a distal end piece (7), from which the laser light (2) is emitted, is arranged on the light-exiting side of the bend (K).

3. Device according to claim 2,
**characterised in**
**that** the capillaries (12) are closed by the distal end piece (7).

4. Device according to one of the claims 2 or 3,
**characterised in**
**that** the exit surface of the distal end piece (7) is formed by grinding and polishing.

5. Device according to one of the claims 1 - 4,
**characterised in**
**that** the optical fibre (4) has several capillary rings (15).

6. Method for the manufacture of a curved optical fibre (4) for a device for medical treatment by means of laser light (2) wherein the optical fibre (4) has a core (9) surrounded by a cladding (10) for conducting the coupled-in laser light (2), wherein the laser light (2) exits from the end face of the light-exiting end (6),
at which the light-exit end (6) of the optical fibre (4) is so curved that the exit direction runs transverse to a largely straight-running section (6a) immediately ahead of the bend (K) section,
**characterised in**
**that** an optical fibre (4) is used that has capillaries (12) running parallel to the fibre longitudinal axis in a cladding (10) at a radial distance from the longitudinal axis (14) [of the optical fibre (4)], seen in cross-section, that form a capillary ring (15) whose capillary cavities (15) are kept separate from each other by webs (17) whose width (18) is less than the wavelength of the laser light (2), and that the capillaries (12) at the light-exit end (6)are closed by melting the light-exit end (6) and that the light-exit end (6) with the closed capillaries (12) is then bent into a hook-shape.

## Revendications

1. Dispositif de traitement médical par lumière laser (2),
avec une fibre optique (4) présentant une âme (9) et une gaine (10) entourant celle-ci pour la transmission de la lumière laser (2) couplée,
où l'extrémité de sortie lumineuse (6) de la fibre optique (4) est courbée et prévue pour la sortie frontale de la lumière laser (2) à l'extrémité de sortie lumineuse (6), la direction de sortie étant transversale à un segment de longueur (6a) situé immédiatement avant la courbure (K) et sensiblement rectiligne,
**caractérisé**
**en ce que** des capillaires (12) s'étendant dans le sens de la longueur de la fibre et radialement espacés de l'axe longitudinal (14) de la fibre optique (4) sont disposés dans la gaine (10), lesquels, vus en section transversale, forment un anneau de capillaires (15) dont les cavités de capillaires (15) sont séparées entre elles par des entretoises (17) dont la largeur (18) est inférieure à la longueur d'onde de la lumière laser (2), et en ce que les capillaires (12) sont obturés par fusion de la fibre optique (4) à l'extrémité de sortie lumineuse (6) de la fibre optique (4), et l'extrémité de sortie lumineuse (6) est coudée en crochet.

2. Dispositif selon la revendication 1,
**caractérisé**
**en ce qu'**un embout (7) distal dont sort la lumière laser (2) est disposé contre la courbure (K) côté sortie lumineuse.

3. Dispositif selon la revendication 2,
**caractérisé**
**en ce que** les capillaires (12) sont obturés par l'embout (7) distal.

4. Dispositif selon la revendication 2 ou la revendication 3,
**caractérisé**
**en ce que** la surface de sortie de l'embout (7) distal est formée par meulage et polissage.

5. Dispositif selon l'une des revendications 1 à 4,
**caractérisé**
**en ce que** la fibre optique (4) comprend plusieurs anneaux de capillaires (15).

6. Procédé de fabrication d'une fibre optique (4) courbée destinée à un dispositif de traitement médical par lumière laser (2),
la fibre optique (4) présentant une âme (9) avec une gaine (10) entourant celle-ci et une extrémité de sortie lumineuse (6) pour la transmission de la lumière laser (2) couplée,
la lumière laser (2) sortant frontalement de l'extrémité de sortie lumineuse (6),
où l'extrémité de sortie lumineuse (6) de la fibre optique (4) est courbée de telle manière que la direction de sortie est transversale à un segment de longueur (6a) situé immédiatement avant la courbure (K) et sensiblement rectiligne,
**caractérisé**
**en ce qu'**une fibre optique (4) est utilisée, laquelle comprend des capillaires (12) s'étendant dans la gaine (10) dans le sens de la longueur de la fibre et radialement espacés de l'axe longitudinal (14) de la fibre optique (4), lesquels, vus en section transversale, forment un anneau de capillaires (15) dont les cavités de capillaires (15) sont séparées entre elles par des entretoises (17) dont la largeur (18) est inférieure à la longueur d'onde de la lumière laser (2), en ce que les capillaires (12) sont obturés par fusion de la fibre optique (4) à l'extrémité de sortie lumineuse (6) de la fibre optique (4), et en ce que l'extrémité de sortie lumineuse (6) avec les capillaires obturés (12) est ensuite coudée en crochet.
